Europäisches Patentamt

(19) European Patent Office

Office européen des brevets

(11) EP 0 678 516 B1

## (12) EUROPEAN PATENT SPECIFICATION

(45) Date of publication and mention
of the grant of the patent:
**27.11.2002 Bulletin 2002/48**

(51) Int Cl.⁷: **C07D 307/56**, A01N 43/08

(21) Application number: **95500056.7**

(22) Date of filing: **21.04.1995**

(54) **Preparation of 2-bromo-5-(2-bromo-2-nitrovinyl)-furan and its use as microcide**

Herstellung von 2-Bromo-5-(2-bromo-2-nitro-vinyl)-furan und deren Verwendung als Microzide

Procédé pour la préparation de 2-bromo-5-(2-bromo-2-nitro-vinyl)-furan et son utilisation comme microcide

(84) Designated Contracting States:
**AT BE CH DE DK ES FR GB GR IE IT LI LU MC NL
PT SE**

(30) Priority: **21.04.1994 CU 4894**

(43) Date of publication of application:
**25.10.1995 Bulletin 1995/43**

(73) Proprietor: **CENTRO DE BIOACTIVOS QUIMICOS
DE LA UNIVERSIDAD CENTRAL DE LAS VILLAS
Santa Clara, Villa Clara (CU)**

(72) Inventors:
 • **Castanedo Cancio, Nilo Ramon
Santa Clara, Villa Clara (CU)**
 • **Goizueta Dominguez, Ramon Donato
Santa Clara, Villa Clara (CU)**
 • **Gonzàlez Garcia, Oraida
Sandino, Santa Clara, Villa Clara (CU)**
 • **Pèrez Donato, Jorge Antonio
Santa Clara, Villa Clara (CU)**
 • **Gonzàlez Feitot, Josefa
Santa Clara, Villa Clara (CU)**
 • **Silveira Prado, Enrique Antonio
Sandino, Santa Clara, Villa Clara (CU)**
 • **Cuesta Mazorra, Mario
Vigia Sur, Santa Clara, Villa Clara (CU)**
 • **Martinez del Pino, Antonio Rafael
Santa Clara, Villa Clara (CU)**

 • **Lugo Farinas, Estela
Rpto Libertad, Santa Clara, Villa Clara (CU)**
 • **Estrada Roger, Ernesto,
Calle 14 escalera 315 Apto
Santa Clara, Villa Clara (CU)**
 • **Carta Fuentes, Ana del Carmen
Las Antillas, Santa Clara, Villa Clara (CU)**
 • **Zarraluqui, Ofelia Navia
Santa Clara, Villa Clara (CU)**
 • **Delgado Lasval, Maria Soledad
Santa Clara, Villa Clara (CU)**

(74) Representative: **Keussen, Christof, Dr.
Glawe, Delfs, Moll & Partner
Patentanwälte
Rothenbaumchaussee 58
20148 Hamburg (DE)**

(56) References cited:
 • **CHEMICAL ABSTRACTS, vol. 76, no. 23, 5 June
1972 Columbus, Ohio, US; abstract no. 139829q,
GRUNTFEST ET AL 'Physicchemical properties
and reactivity of furylnitroolefins.' page 390;
column 2; & ZH.ORG.KHIM, vol. 8, no. 2, 1972
pages 404-411,**
 • **CHEMICAL ABSTRACTS, vol. 78, no. 7, 19
February 1973 Columbus, Ohio, US; abstract no.
43166m, NAZAROVA ET AL 'Synthesis of some
furylnitro olefins with potential biological
activity' page 463; column 1; & KHIM.FARM.ZH.,
vol. 6, no. 10, 1972 pages 5-8,**

**Description**

The following invention relates human and animal health in general with a product which has an extensive microcide activity, and which has been evaluated "in vitro", in experimental and stock animals and in humans with a wide number of gram positive bacteria (more than 500 strains), gram negative (more than 1500 strains), fungi (more than 500 strains) and other microorganisms (more than 100 strains).

This substance was described in the international literature by author Z.N. Nazarova during 1972. The first time was in an article "Synthesis of some furyl-nitro-olefins with potential biological activity" together with the author G.F. Potemkin in the review Khim. Farm. Zh. 6 (10); 5-8 (1972), the second in an article "Physicochemical properties and reactivity of furyl-nitro-olefins" together with authors M.G. Gruntfest, G.F. Potemkin, Yu. V. Kolodyazhnyi, V. Zverev and O.A. Osipov in the review Zh. Org. Khim 8(2); 404-11 (1972).

In the first article the synthesis routes used by the author were covered and in the second chemical-physical studies of a wide range of substituted furyl-nitro-olefins were described, and the calculation of dipolar moments, some data from infra-red and ultraviolet spectra and certain theoretical studies about these systems were included.

Neither of the two articles published reported any specific biological actions of the product.

The obtention procedures described by the author in the first article are the following:

A solution of 0.03 moles of bromine in 20 ml of chloroform is added to a solution of 0.015 moles of β-furyl-nitro-olefins in 50 ml of chloroform with constant agitation and a temperature of 0°C for 30 minutes.

The solution is left for 3-4 hours, at a temperature of 20-25°C, after which the solvent is distilled at room temperature. The residue obtained is β-(5-bromofur-2-yl)-α,β-dibromo-nitroethane in the form of a pale yellow oil, which decomposes if stored or distilled.

A solution of 3.4 g of potassium hydroxide in 50 ml of water with energetic shaking and a temperature of 20-25°C was added to 5.3 g of the oil obtained.

The precipitate formed is separated, washed with water to obtain a neutral medium, dried in the drier and then recrystallized twice in hexane. The reported yield was 27 g (64%), melting temperature of 94-95°C, yellow crystals.

Another proposed method consists in:

To a solution of 0.01 moles of 5-bromofurfural and 0.01 moles bromonitromethane in 30 ml of methanol chilled in a mixture of ice and salt, one adds, drop by drop, whilst shaking constantly for 30 minutes, a solution of 0.02 moles of potassium hydroxide in 3 moles of water and little by little the cold mixture is poured into 3.2 ml of concentrated hydrochloric acid and 12 ml of water. After 30 minutes the precipitate is separated, washed with water to obtain a neutral medium, purified with a steam washed distillation and subsequently recrystallized with alcohol.

The reported yield in this case was 76.8%, with a melting temperature of 88-89°C.

The author states that the product obtained by both routes are identical, bearing in mind the infra-red (IR) and ultraviolet (UV) spectrums and the thin layer chromatography. The invention is defined in the claims.

The proposed procedure for making the compound used in the present invention produces greater yield and higher purity rates and the same consists in the reaction of 2-(2-nitro-vinyl)-furan, with bromine in a molar ratio, which oscillates between 1:2 and 1:2.6, using non polar solvents such as: carbon disulphide or carbon tetrachloride, being shaken between 1 and 6 hours, and controlling the temperature between 20 and 60°C.

The solvent is then separated by simple atmospheric pressure distillation, the majority of the possible excess bromine is eliminated during this operation, as well as part of the hydrogen bromide formed.

Subsequently, both neutralization and dehydrobromination is carried out, simultaneously, using a tertiary amine, and a temperature control in the range of 20-70°C.

At the end of this stage of the experiment the product precipitates into a yellow mass, which is dissolved in a hot polar solvent and the same precipitates from the solution after some hours of resting.

Yields higher than 90% are achieved on the scales reported by Z.N. Nazarova and in the scale of 5.5 moles never less than 63%. The purity obtained in small scales are always higher than 90% and in the larger scales never less than 75%, the reduction being caused by the fact that up until now a total drying process has not been completed on doing analysis.

The purity of the raw product is determined by gas chromatography and HPLC.

The crude product undergoes a purification process which consists in dissolving it in a polar solvent such as: ethanol, isopropanol or methanol at a temperature of between 25 and 70°C and immediately adding a finely divided adsorbent product and maintaining it in agitation for 10 to 60 minutes.

It is then filtered, the solution is left to rest for some hours and the product finally crystallizes into yellow crystals which are separated by filtration, dried and packed.

The pure product is of a purity which oscillates between 98 and 100%, the established limit by pharmacopoeia for using active principles as medicaments.

The main impurity which appears in the synthesis is the 2- bromo-5-(2-nitro-vinyl)-furan and its content oscillates between 0.1 and 1.99%.

The product obtained is thus in the form of yellow crystals and has a melting temperature of 88-89°C.

The advantages of this new procedure are varied and stem from the fact that the complete synthesis process can be carried out in an organic phase which avoids losses and greatly reduces the high number of operations needed by the presence of the water phase. This has made it possible to scale the reaction starting from the scale of 0.015 moles reported by Z.N. Nazarova to 5.5 moles, i.e. a scale which is 366 times greater, good results being obtained; the use of tertiary amine means that the pH of the reaction medium can be neutralized simultaneously and the dehydrobromination reaction can be carried out without resins forming, which had always occurred with the method described in the literature. Z.N. Nazarova does not report on the purity of the product obtained after purification, and what is striking is that the results obtained in the condensation of 5-bromofurfural with bromonitromethane are 76.8% and the reported melting temperature is 88-89°C and by means of bromination of the 2-(2-nitrovinyl)furan the result is 64% and the melting temperature is 94-95°C, in spite of always obtaining a mixture of the two components. On reproducing this technique in a laboratory, using the latter method in the same scale described by the author, apart from obtaining very low yield, there always appeared to be a considerable concentration of impurity described by us, i.e. of 2-bromo-5-(2-nitro-vinyl)-furan. Using the procedure described by ourselves, the purity obtained is always guaranteed, to be above 98% and the limits of impurity previously described are maintained below 2%, which means, on applying good production practices, a reliable active principle may be supplied for the creation of finished medicative forms in human and veterinary medicine.

The component obtained using the procedure described in this invention shows a mass spectrum with peaks in m/e=297, corresponding to the molecular ion and also shows peaks M+2 and M+4 characteristic for the presence of two atoms of bromine in the structure. This peak (M$^+$=297) corresponds to the global formula $C_6H_3NO_3Br_2$, which also fulfills the nitrogen rule.

In the IR spectrum of this compound, registered in solid state (KBr disc, the bands of the nitro group appear at 1507 cm$^{-1}$ and 1306 cm$^{-1}$ and correspond to the antisymmetrical and symmetrical stretching vibration of the mentioned group. Another band of interest is that which appears at 1618 cm$^{-1}$, corresponding to the stretching vibrations of Carbon-Carbon double bonds. The presence of a high electronic conjugation in the molecule is proven by the UV spectrum where a band of great intensity appears (lgE=4.09) at 371 nm when the spectrum is registered using a methanolic solution. The assignation of this band corresponds to a transition $\pi \to \pi^*$ was undertaken with the register of the UV spectrum in solvents of different polarities where a notable hypsochromic shift occurred when the dielectric constant of the medium was reduced.

The $^1$H-NMR spectrum was registered using $CDCl_3$ as a solvent and only three signals with chemical shifts of 6.63, 7.39 and 8.50 ppm were observed, each one with the same relative intensity, which corresponds to a single proton, when the overall formula is taken into consideration.

The assignations of these signals are carried out using the tables of additive increments for chemical shifts. The values calculated were of 6.59 for a proton linked to carbon 3 of the furan ring. The chemical shift which appears at 8.50 ppm is characteristic for a high shielded olefin proton. From the calculation of the chemical shift for a proton linked to a double bond with a nitro and a bromine in β-position, it could be determined that this proton stays in trans position with respect to the bromine and in cis position with respect to the nitro, corresponding to a Z-configuration for the substituting groups (chemical shift for Z is 8.48 and for E is 7.70 ppm).

All the carbon atoms in the molecules from the $^{13}$C-NMR spectrum were expected to be of sp2 type, as was expected from the previous data.

The crystallographic study was carried out for this compound determining that the same had an ortorhomboidal structure and the spacial configuration was checked for the previously established atoms. From this complete structural analysis it was concluded that the compound in question is the (Z)-2-bromo-5-(2-bromo-2-nitro-vinyl)-furan.

The antimicrobial sensitivity was first evaluated "in vitro" using the serial dilution method for bacteria and fungi. Other growth methods in agar cultures were included for other species. The liquid and solid mediums used were Mueller-Hinton, Tryptona Soya, Sabouraud Dextrosa, Sabouraud Maltose and other mediums in accordance with the cultural demands of the micro-organisms valued.

The temperature and incubation period was adjusted to conditions and characteristics of the micro-organisms used.

The Minimal Inhibitory Concentration (MIC) was calculated in terms of $MIC_{50}$ and $MIC_{90}$ in µg/ml.

EXAMPLES OF OBTENTION PROCEDURE

Example 1

In the following table some examples of synthesis are presented according to the procedure proposed, using the same scale reported by Z.N. Nazarova.

| Molar relation | Time (h) | Temperatures (°C) | Yield (%) | Purity (%) |
|---|---|---|---|---|
| | | | | |
| 1:2 | 4 | 50 | 80.4 | 98.0 |
| 1:2:5 | 3 | 45 | 85.0 | 98.8 |
| 1:2:5 | 4 | 40 | 93.0 | 99.5 |
| 1:2:5 | 5 | 50 | 86.2 | 98.2 |

Some results using a scale 366 times higher than the published in the literature were reported.

Example 2

| No. Synthesis | Raw Yield (%) | %Purity Pure Product | %Impurity |
|---|---|---|---|
| 1 | 63.05 | 98.34 | 1.66 |
| 2 | 66.11 | 98.99 | 1.01 |
| 3 | 71.01 | 98.65 | 1.35 |
| 4 | 66.72 | 98.81 | 1.19 |
| 5 | 71.93 | 99.54 | 0.46 |
| Note: The quantitative purity was obtained with gas chromatography. | | | |

Example 3

IN VITRO ASSAYS CONDUCTED WITH THE CORRESPONDING VALUES OF MINIMAL INHIBITORY CONCENTRATIONS (MIC) AGAINST THE FOLLOWING MICROORGANISMS.

1. Of importance in human and animal health
    $MIC_{50}$ values        $MIC_{90}$ values
     μg/ml        μG/ml
Bacteria

   1.1 Gram Negative

       1.1.1. Family: Pseudomonadaceae

| Pseudomonas aerguinosa | 07.84 | 14.97 |
|---|---|---|
| Pseudomonas maltophila | 29.73 | 45.06 |

       1.1.2. Family: Enterobacteriaceae

| Escherichia coli | 07.53 | 15.97 |
|---|---|---|
| Enterobacter spp. | 05.30 | 17.16 |
| Klebsiella spp. (including K. pneumoniae) | 10.99 | 21.34 |
| Proteus mirabilis | 9.36 | 24.15 |
| Proteus vulgaris | 05.95 | 19.75 |
| Proteus morganii | 03.13 | 10.15 |
| Proteus rettgeri | 02.78 | 09.92 |

(continued)

| Enterobacter aglomerans | ("in vivo" tests) | |
|---|---|---|
| Salmonella typhimurium | 07.43 | 17.68 |
| Salmonella enteritidis | 06.86 | 11.50 |
| Salmonella oxford | 04.42 | 05.83 |
| Salmonella dublin | 04.38 | 05.73 |
| Salmonella choleraesuis | 05.57 | 10.51 |
| Salmonella thyphi | 02.50 | 10.00 |
| Serratia marcescens | 05.03 | 10.95 |

1.1.3. Family: Brucellaceae

| Pasteurella haemolytica | 03.72 | 05.63 |
|---|---|---|
| Pasteurella multocida | 03.59 | 09.47 |
| Haemophilus influenzae | 03.51 | 39.23 |
| Moracella bovis | 02.63 | 05.08 |

1.1.4. Family: Neisseriaceae

| Neisseria meningitidis | 01.84 | 03.47 |
|---|---|---|

1.2. Gram Positive

1.2.1. Family: Micrococaceae

| Staphylococcus aureus | 05.91 | 12.94 |
|---|---|---|
| Staphylococcus albus | 10.25 | 19.84 |
| Sarcina lutea | 01.56 | 04.43 |

1.2.2. Family: Lactobacillaceae

| Streptococcus agalactiae | 01.99 | 04.55 |
|---|---|---|
| Streptococcus pyogenes | 01.86 | 04.42 |

1.2.3. Family: Corynebacteriaceae

| Corynebacterium pyogenes | 03.56 | 05.59 |
|---|---|---|
| Listeria monocytogenes | 03.13 | 05.44 |

1.2.4. Family: Bacillaceae

| Bacillus subtilis | 02.27 | 05.08 |
|---|---|---|
| Bacillus alvei | 02.21 | 05.08 |

1.3. Superiors

1.3.1. Family: Treponemataceae

| Leptospira canicola | 09.36 | 15.39 |
|---|---|---|
| Leptospira pomona | 08.84 | 11.66 |

### 1.3.2. Family: Spirillaceae

| | | |
|---|---|---|
| Vibrio cholerae ser. Inabu | 05.87 | 17.68 |

### 1.4. Escotobacterias

#### 1.4.1. Family: Mycoplasmataceae
Mycoplasma bovigenitalium 50.00
Fungi

### 1.5. Monomorphic budding fungi

#### 1.5.1. Family: Cryptococcaceae

| | | |
|---|---|---|
| Candida albicans | 03.11 | 07.95 |
| Candida parasilosis | 03.56 | 06.93 |
| Candida tropicalis | 02.78 | 05.29 |
| Candida pseudotropicalis | 01.92 | 03.56 |
| Candida zeyfanoide | 01.92 | 03.56 |
| Candida glabrata | ("in vivo" tests) | |
| Candida stellatoidea | 01.92 | 03.56 |
| Candida guilliermondi | 01.92 | 02.84 |
| Candida krusei | 01.92 | 02.84 |
| Candida sp. | ("in vivo" tests) | |
| Malassezia furfur | ("in vivo" tests) | |

### 1.6. **Monomorphic Filamentous Fungi**

#### **1.6.1. Family:** Moniliaceae

| | | |
|---|---|---|
| Aspergillus sp. | 16.21 | 48.30 |
| Trichophyton rubrum | 13.80 | 24.03 |
| Trichophyton mentagrophytes | 17.68 | 40.61 |
| Microsporum canis | 07.91 | 40.61 |
| Microsporum gypseum | 12.50 | 20.78 |
| Epidermophyton floccasum | 12.50 | |

#### **1.6.2. Family:** Tuberculariaceae

| | |
|---|---|
| Fusarium sp. | 50.00 |

Reproduction and experimental treatment studies have been carried out for bacterial and fungus infections in various laboratory animal species such as: rats, mice, guinea-pigs, hamster, rabbits and dogs, using different formulations of the active principle which is the 2-bromo-5-(2-bromo-2-nitro-vinyl)-furan.

In toxicological studies in animals of experimentation, necessary to guarantee effectiveness studies about which reference is made in this descriptive record, it was proven that the Lethal Average Dose ($LD_{50}$) taken orally of 2-bromo-5-(2-bromo-2-nitro-vinyl)-furan using a tragacant gum as the vehicle was of 1731.4 and 1835.8 mg/kg of body mass for mice OF1 females and male, respectively and of 1515.5 and 1857 mg/kg of body mass for female and male Sprague Dawley rats respectively. By percutaneous route, applying the product in the interscapular region (approximately in an area of 10% of the animal) the quantities of 0.2 ml/10 g of body mass for mice OF1, the $LD_{50}$ was 9.8% and 12.1% in females and males, respectively, and 14.7 and 15.8% for female and male Sprague Dawley rats, respectively, applying the product in the interscapular region of 0.2 ml/10 g of body mass. Moreover, the anatomopathological elements and the clinical events were characterized during the experiments. In ophthalmic irritability studies it was proven that for concentrations lower than 1.25% no irritating effects were made on the eye structure.

Chronic studies of 2-bromo-5-(2-bromo-nitro-vinyl)-furan were made on Sprague Dawley rats. The haematological, haemochemical and histopathological studies revealed that the target product applied externally for a period of six months did not lead to any major problems in the different organs and tissues analysed.

**Example 4**

The "in vivo" effectiveness of four ointments of 2-bromo-5-(2-bromo-2-nitro-vinyl)-furan was evaluated at 1, 0.5, 0.25 and 0.125% in hydrophislous ointment applying it on 2 cm$^2$ of burns provoked to guinea-pigs and subsequently infected with Pseudomonas aeruginosa by a burn wound in an area of 2 cm$^2$, using positive and negative control groups. It was found that the effective dose 50 (ED$_{50}$)was achieved with the concentration of 0.125% (Table N°1).

Table 1.  Effectiveness of Product G-1 in skin infections by aeruginous P.

| Group | N | Concent. G-1 | Days tto. | Deaths Days (*) No | | Isolation in organs L S K L H B | Survival | Observation |
|---|---|---|---|---|---|---|---|---|
| I | 10 | 1 g% | 7 | 11 | 1 | + + + + + + | 90.0 | Sign of irritation in wound |
| II | 10 | 0.5 g% | 7 | 10 | 1 | + + + + + + | 90.0 | |
| III | 10 | 0.25 g% | 7 | 9 | 1 | + + + + + + | 90.0 | |
| IV | 10 | 0.125g% | 7 | 14<br>15 | 2<br>1 | 2+ 2+ 2+ 2+ 2+ 2+<br>+ + + + + + | 70.0 | Normal |
| V | 10 | Control | – | 9<br>15<br>19<br>21 | 1<br>1<br>4<br>2 | + + + + + +<br>+ + + + + +<br>4+ 4+ 4+ 4+ 4+ 4+<br>2+ 2+ 2+ 2+ 2+ 2+ | 20.0 | Inflammations, secretion, diarrhoea, microabscess Generally poor state |

Read:

L=Liver; S= Spleen; K= Kidney; L=lung, H= Heart, B=Brain.

G-1 = refers to 2-bromo-5-(2-bromo-2-nitro-vinyl)-furan

(*) = Day on which the animal died after inoculation

Example 5

The effectiveness of two oil formulations was checked of 0.05% for the experimental treatment of eye infections by Pseudomonas aeruginosa in New Zealand Rabbits through the erosion and instillation method. The effectiveness of treatments was tested clinically and microbiologically, observing significant differences with the control group (not treated) (Table No. 2 and 2.1.).

Table 2:

| Post inoculation clinical and microbiological evaluation | | | | | |
|---|---|---|---|---|---|
| Group | Secretions | Hyperemia | Edam | Ulcer | Bacteriolo-gical Exam |
| I | 6/6 | 6/6 | 6/6 | 6/6 | 6 (+) |
| II | 6/6 | 6/6 | 6/6 | 6/6 | 6 (+) |
| III | 6/6 | 6/6 | 6/6 | 6/6 | 6 (+) |
| IV | 6/6 | 6/6 | 6/6 | 6/6 | 6 (+) |
| Legend:<br>I. Treated with ointment of 2-bromo-5-(2-bromo-2-nitro-vinyl)-furan at 0.05% for 8 days<br>II. Treated with ointment of 2-bromo-5-(2-bromo-2-nitro-vinyl)-furan at 0.05% for 4 days<br>III. Treated with eye drops of 2-bromo-5-(2-bromo-2-nitro-vinyl)-furan at 0.05% for 4 days<br>IV. Control (inoculated without treating) | | | | | |

The effectiveness of three oil formulations were also tested, at 0.15, 0.2, 0.25% and a control with placebo in mice experimentally infected using implanted sponge method, inoculated with Pseudomonas aeruginosa and Staphylococcus aureus. Recuperation of the animal was between 3 and 4 days of treatment (Tables No. 3 and 3.1).

## Table 3. Effectiveness of treatment in mice inoculated with Staphylococcus aureus

| Group | Formula (%) | n | Average weight | Sex | Accumulated clinical recuperation (days) | | | | | | | | Tr (days) |
|---|---|---|---|---|---|---|---|---|---|---|---|---|---|
| | | | | | 1 | | 3 | | 5 | | 7 | | |
| | | | | | n | % | n | % | n | % | n | % | |
| I | 0.15 | 9 | 26.5 | M | 1 | 11.1 | 6 | 66.7ab | 8 | 88.9ab | 9 | 100.0 | 3.7 |
| II | 0.2 | 9 | 26.7 | M | 1 | 11.1 | 8 | 88.9a | 9 | 100.0a | 9 | 100.0 | 3.0 |
| III | 0.25 | 9 | 27.3 | M | 1 | 11.1 | 5 | 55.6ab | 9 | 100.0a | 9 | 100.0 | 3.7 |
| IV | 0 Control | 9 | 27.7 | M | | | 3 | 33.8bc | 7 | 77.8bc | 9 | 100.0 | 4.8 |

Proportions with letters which are not common differ statistically for (p< 0.05).
Read:

n =      Accumulated from recuperated animals
Tr =     Recuperation time in days (weighted average)

$$((n1\pm1)+((n2-n1)\pm2)+((n3-n2)\pm3((n4-n3)\pm4)/n$$

I, II, III: treated with 2-bromo-5-(2-bromo-2-nitro-vinyl)-furan.

Tab. 3.1.

| Effectiveness of treatment in mice inoculated with Pseudomonas aeruginosa. | | | | | | | | | | | | |
|---|---|---|---|---|---|---|---|---|---|---|---|---|
| Group | Formula (%) | n | Average Weight | Sex | Accumulated clinical recuperation (days) | | | | | | | I Tr (days) |
| | | | | | 3 | | 5 | | 7 | | | |
| | | | | | n | % | n | % | n | % | | |
| I | 0.15 | 10 | 22.6 | H | 8 | 80.0a | 10 | 100.0a | 10 | 100.0 | | 3.43.4 |
| II | 0.2 | 10 | 22.9 | H | 8 | 80.0a | 10 | 100.0a | 10 | 100.0 | | 4.24.6 |
| III | 0.25 | 10 | 22.8 | H | 5 | 50.0a | 9 | 90.0a | 10 | 100.0 | | |
| IV | 0 Control | 10 | 23.6 | H | 4 | 40.0a | 8 | 80.0bc | 10 | 100.0 | | |

Proportions with letters which are not common differ statistically for (p< 0.05).
Read:

n =     Accumulated from recuperated animals
Tr =     Recuperation time in days (weighted average)

$$((n1\pm1) + ((n2\text{-}n1) \pm2)+((n3\text{-}n2)\pm3((n4\text{-}n3)\pm4)/n$$

I,II, III: treated with 2-bromo-5-(2-bromo-2-nitro-vinyl)-furan.

**Example 6**

The effectiveness of the active principle against bacterial eye diseases in: 9435 animal eyes affected with queratoconjunctivitis; 8093 of cattle and 1342 of other species (sheep, rabbits, dogs, horses, pigs, cats, guinea-pigs and birds). The results showed a rising clinical recuperation to 89,6%, oscillating between 74 and 91% (Table No 4).
In some cases the result of the medicament was compared with others habitually used for these treatments.

Table 4:

| Summary of results of clinical tests of G-1 ointments in the external treatment of eye infections in animals. | | | | | | | | | | | | | |
|---|---|---|---|---|---|---|---|---|---|---|---|---|---|
| Species | Sufferings | | | | Applied | | | formulations | | | | | |
| | | G-1 ointment | | | Queraforte spary | | | Chloran-fenicol-(U) | | | Chloran-fenicol-(I) | | |
| | | n | P± | Re(%) | n | P± | Re (%) | n | P± | Re (%) | n | P± | Re (%) |
| Cows (PE) | 1 | 36 | 2.9a | 100.0a | 36 | 3.2ab | 97.2ab | - | - | - | 36 | 3.1ab | 80.6ba |
| Dogs | 2,3,4, | 91 | 2.9a | 93.4a | 20 | 5.4c | 25.0c | 28 | 7.3b | 81.5a | - | - | - |
| Rabbits | 2 and 5 | 42 | 3.0a | 88.1a | 29 | 3.5a | 80.6a | 22 | 3.2a | - | - | - | - |
| Sheep | 3 | 12 | 3.4a | 75.0a | 11 | 3.7a | 72.7a | - | - | - | - | - | - |
| Horses | 2 and 4 | 46 | 3.7a | 95.7a | 22 | 4.2b | 90.9a | - | - | - | - | - | - |
| Pigs | 2 and 4 | 16 | 3.6a | 75.0a | 13 | 3.8a | 76.9a | - | - | - | - | - | - |
| Cats | 2 | 10 | 3.2a | 90.0a | 8 | 3.4a | 87.5a | - | - | - | - | - | - |
| Cattle (E) | 1 | 1830 | 3.1a | 85.0a | 285 | 3.1a | 82.1a | 164 | 3.4a | 86.6a | - | - | - |
| Guinea-pig | 6 | 27 | 4.3 | 74.1b | - | - | - | - | - | - | - | - | - |
| Birds | >> | 21 | 2.8 | 85.7a | - | - | - | - | - | - | - | - | - |
| (*) | >> | 6124 | | 89.60 | - | - | - | - | - | - | - | - | - |

Proportions with letters not in common differ statistically for (p< 0.01)

Read:

1 = Bovine infectious queratoconjunctivitis

(PE) = small scale or clinical test in conditions controlled on a small scale

(U) = ointment

(I) = injectable

n = number of eyes treated

P± = Average of treatment for clinical recuperation (weighted average)

Rc = clinical recuperation

2 = conjunctivitis

3 = Querratitis

4= Queratoconjunctivitis

5= Blefaritis

6= general eye infection

7= clinical extension or test compared in conditions of extension

(*) = Massive application in therapeutic survey in the new different animal species

(G-1) = refers to 2-bromo-5-(2-bromo-2-nitro-vinyl)-furan

## Example 7

The active principle was administered in a dose of 5-10 mg/kg of body mass by intramuscular route in bulls affected with piocianosis Pseudomonas aeruginosa. The animals clinically recuperated without any allergic reactions to the medicament being detected. Bactericide levels in serum were tested from 1 to 24 hours post-treatment and the bacteriological tests carried out in the semen at 3,7,14,21 and 28 days were negative at germ, no alterations being detected in seminal parameters established as quality control in the establishments of artificial insemination of the country. In clinical tests no allergic reactions were detected in animals (Table No.5).

Subsequent studies carried out with frozen semen of treated bulls demonstrated the absence of cytotoxicity and variations in morphological characteristic of spermatozoid.

Table 5:

| Effectiveness of treatment with 2-bromo-5-(2-bromo-2-nitro-vinyl)-furan on Artificial Insemination Stud Bulls affected by Pseudomonas aeruginosa. | | | |
|---|---|---|---|
| No. Ill animals | No. Treatments per animal | Recuperated | percentage of effectiveness |
| 25 | 5 | 21 | 84% |
| Legend: G-1 = refers to 2-bromo-5-(2-bromo-2-nitro-vinyl)-furan | | | |

## Example 8

The active principle in a concentration of 0.1 % is applied by intracisternal route daily for three consecutive days in femal oxes clinically affected by mastitis of morbid catarrh purulen course types, in which the isolated microorganisms were Streptococcus agalactiae and Staphylococcus aureus. As a control medicament the mammary infusion of neomicine was used.

Clinical recuperation of the affected parts was 98.25% for the infusion of G-I and 100% for the control medicament (Table No. 6).

Table 6:

| Clinical recuperation of the affected parts of clinical mastitis | | | |
|---|---|---|---|
| Treatment | Recuperated | | Total |
| | n | % | |
| G-I | 56 | 98.25 | 57 |
| Neomicine | 12 | 100 | 12 |
| Totals | 68 | 98.55 | 69 |

Table 6: (continued)

| Clinical recuperation of the affected parts of clinical mastitis | | | |
|---|---|---|---|
| Treatment | Recuperated | | Total |
| | n | % | |
| Legend: G-1 = refers to 2-bromo-5-(2-bromo-2-nitro-vinyl)-furan | | | |

**Example 9**

The active principle was used in an oil solution at 1.5% for the treatment of septic puerperal metritis in female oxes. The treatment consisted in applying 40 ml for three consecutive days at intervals of 24 hours from the diagnosis of the disease process. Treatment was compared with intrasoric tetracyclin.

27 females were treated with a total of 20 (74.1%) with total recuperation with an average clinical treatment-recuperation interval (T-R) of 17.7 days. With the tetracycline, of the 19 animals treated, 13 recuperated (68.4%) and the T-R was 39 days. Indices of insemination were $1.09 \pm 0.30$ for the new formula and of $1.78 \pm 1.99$ days for the tetracycline with significant statistical differences ($p < 0.01$) (Table No. 7).

Table 7:

| Clinical recuperation of septic puerperal metritis using G-1. | | | | | |
|---|---|---|---|---|---|
| Antibiotic Application route | Total females treated | Recuperated n | % | T-R | IIA |
| G-1 at 1.5% oil solution Intrauterine route | 27 | 20 | 74.1 | 17.7 | 1.091±0.30a |
| Tetracycline Abdominal aorta route | 19 | 13 | 68.4 | 39 | 1.78± 1.99b |

Proportions with letters no in common differed statistically for ($p < 0.01$).

Legend:
T-R = Average of treatment interval of clinical recuperation          HA = Artificial insemination index          G-1 = Refers to 2-bromo-5-(2-bromo-2-nitro-vinyl)-furan.

**Example 10**

A cream was developed which was subjected to clinical tests in Phase III in 386 patients in four hospitals. In the first piodermitis effectiveness was of 89.2% comparable with that of Gentamicin. In the candidiasis in skin effectiveness was 79.3% in versicolour pityriasis of 88.0% in dermatomycosis of 76.9% in onicomicosis by Candida of 32.6% and in onicomicosis by dermatophitos 42.9%, its effectiveness being comparable to that of Ketoconazol.

The studies on the active principle and pharmaceutical forms indicate that they combine a strong antibacterial and antifungal action of wide spectrum, with a toxicity similar to that presented by many medicaments which may currently be found on the market (Tables No.8 and 8.1).

Table 8:

| Effectiveness of treatment | | | | | | |
|---|---|---|---|---|---|---|
| Pathologies | | G-1 cream | | | Control | |
| | a | b | % Patients | a Cured | b | % |
| Primary Piodermitis | 37 | 33 | 89.2 | 37 | 36 | 97.3 |
| Versicolour pityriasis | 25 | 22 | 88.0 | 30 | 26 | 86.7 |
| Skin candidiasis | 29 | 23 | 79.3 | 28 | 24 | 85.7 |
| Dermatomycosis | 39 | 30 | 76.9 | 31 | 29 | 93.5 |
| Onicomicosis by Candida | 46 | 15 | 32.6 | 30 | 14 | 46.7 |
| Onicomocosis by dermatofitos | 28 | 12 | 42.9 | 26 | 8 | 30.8 |
| Cured and improved | | | Patients | | | |
| Primary Piodermitis | 37 | 34 | 91.9 | 37 | 37 | 100.0 |

Table 8: (continued)

| Effectiveness of treatment | | | | | | |
|---|---|---|---|---|---|---|
| Pathologies | | G-1 cream | | | Control | |
| Cured and improved | | | Patients | | | |
| Versicolour pityriasis | 25 | 24 | 96.9 | 30 | 28 | 93.3 |
| Skin candidiasis | 29 | 25 | 86.2 | 28 | 24 | 85.7 |
| Dermatomycosis | 39 | 34 | 87.2 | 31 | 29 | 93.5 |
| Onicomicosis by Candida | 46 | 33 | 71.7 | 30 | 20 | 66.7 |
| Onicomocosis by dermatofitos | 28 | 20 | 71.4 | 26 | 12 | 46.2 |
| Legend:<br>a = Total of cases included<br>b = Patients recuperated<br>G-1 = Refers to the 2-bromo-5-(2-bromo-2-nitro-vinyl)-furan | | | | | | |

Table 8.1:

| Stay in days of cured patients (average) | | |
|---|---|---|
| Pathologies | Cream G-1 | Control |
| Primary Piodermitis | 52.2 | 43.1 |
| Versicolour pityriasis | 50.9 | 57.6 |
| Skin candidiasis | 120.4 | 120.9 |
| Dermatomycosis | 126.1 | 124.6 |
| Onicomicosis by Candida | 167.7 | 291.4 |
| Onicomicosis by dermatofitos | 253.2 | 305.7 |

Read:
       G-1 = Refers to the 2-bromo-5-(2-bromo-2-nitro-vinyl)-furan

## Claims

1.   2-Bromo-5-(2-bromo-2-nitro-vinyl)-furan for use as medicament in human and veterinary medicine.

2.   2-Bromo-5-(2-bromo-2-nitro-vinyl)-furan as claimed in claim 1, wherein the purity of this compound is at least 98 %.

3.   Use of 2-bromo-5-(2-bromo-2-nitro-vinyl)-furan for the manufacture of a medicament for the treatment of microbian infections caused by gram negative, gram positive bacteria, superior bacteria and scotobacteria.

4.   Use of the 2-bromo-5-(2-bromo-2-nitro-vinyl)-furan, according to claim 3, for the manufacture of a medicament for the treatment of infections caused by gram negative bacteria from genera of the family Pseudomonadaceae such as Pseudomonas.

5.   Use of the 2-bromo-5-(2-bromo-2-vitro-vinyl)-furan, according to claim 3, for the manufacture of a medicament for the treatment of infections caused by gram negative bacteria from genera of the family Enterobacteriaceae such as Escherichia, Proteus, Salmonella, Klebsiella, Serratia and Enterobacter.

6.   Use of the 2-bromo-5-(2-bromo-2-nitro-vinyl)-furan, according to claim 3, for the manufacture of a medicament for the treatment of infections caused by bacteria from aerobic and microaerophile genera of the familiy Brucellaceae such as Pasteurella, Haemophilus and Moraxella.

7.   Use of the 2-bromo-5-(2-bromo-2-nitro-vinyl)-furan, according to claim 3, for the manufacture of a medicament for the treatment of infections caused by bacteria from genera of the family Neisseriaceae such as the genus Neisseria.

8. Use of the 2-bromo-5-(2-bromo-2-nitro-vinyl)-furan, according to claim 3, for the manufacture of a medicament for the treatment of infections caused by bacteria from genera of the family Micrococcaceae such as Staphylococcus and Sarcina.

9. Use of the 2-bromo-5-(2-bromo-2-nitro-vinyl)-furan, according to claim 3, for the manufacture of a medicament for the treatment of infections caused by bacteria from genera of the family Lactobacillaceae such as the genus Streptococcus.

10. Use of the 2-bromo-5-(2-bromo-2-nitro-vinyl)-furan, according to claim 3, for the manufacture of a medicament for the treatment of infections caused by genera of asporogenous gram positive bacilli of the family Corynebacteriaceae such as Corynebacterium and Listeria.

11. Use of the 2-bromo-5-(2-bromo-2-nitro-vinyl)-furan, according to claim 3, for the manufacture of a medicament for the treatment of infections caused by genera of endosporogenous gram positive bacilli of the family Bacillaceae such as the genus Bacillus.

12. Use of the 2-bromo-5-(2-bromo-2-nitro-vinyl)-furan, according to claim 3, for the manufacture of a medicament for the treatment of infections caused by genera of the higher bacteria from the family Treponemataceae such as Leptospira.

13. Use of the 2-bromo-5-(2-bromo-2-nitro-vinyl)-furan, according to claim 3, for the manufacture of a medicament for the treatment of infections caused by genera of the higher bacteria from the family Spirillaceae such as Vibrio.

14. Use of the 2-bromo-5-(2-bromo-2-nitro-vinyl)-furan, according to claim 3, for the manufacture of a medicament for the treatment of infections caused by Scotobacteria from the family Mycoplasmataceae.

15. Use of the 2-bromo-5-(2-bromo-2-nitro-vinyl)-furan, according to claim 1, for the manufacture of a medicament for the treatment of infections caused both by budding monomorphic fungi and filamentous monomorphic fungi.

16. Use of the 2-bromo-5-(2-bromo-2-nitro-vinyl)-furan, according to claim 15, for the manufacture of a medicament for the treatment of infections caused by budding monomorphic fungi from the family Cryptococcaceae such as Candida and Malassezia.

17. Use of the 2-bromo-5-(2-bromo-2-nitro-vinyl)-furan, according to claim 15, for the manufacture of a medicament for the treatment of infections caused by filamentous monomorphic fungi including dermatophytes.

18. Use of the 2-bromo-5-(2-bromo-2-nitro-vinyl)-furan, according to claim 15, for the manufacture of a medicament for the treatment of infections caused by filamentous monomorphic genera from the family Moniliaceae such as Aspergillus, Trichophytum, Microsporum and Epidermophytum.

19. Use of the 2-bromo-5-(2-bromo-2-nitro-vinyl)-furan, according to claim 15, for the manufacture of a medicament for the treatment of infections caused by filamentous monomorphic genera from the family Tuberculariaceae such as Fusarium sp.

20. A pharmaceutical composition containing the active substance 2-bromo-5-(2-bromo-2-nitro-vinyl)-furan, according to claim 1, in an appropriate dosage form.

21. The pharmaceutical composition of claim 20, wherein the composition contains a carrier, excipient, diluent or adjuvant.

## Patentansprüche

1. 2-Brom-5-(2-brom-2-nitrovinyl)furan als Arzneimittel in der Human- und Tiermedizin.

2. 2-Brom-5-(2-brom-2-nitrovinyl)furan nach Anspruch 1, wobei die Reinheit dieser Verbindung mindestens 98% beträgt.

3. Verwendung von 2-Brom-5-(2-brom-2-nitrovinyl)furan zur Herstellung eines Arzneimittels zur Behandlung von Mikroorganismeninfektionen, die durch gramnegative Bakterien, grampositive Bakterien, höhere Bakterien und Scotobakterien verursacht werden.

4. Verwendung von 2-Brom-5-(2-brom-2-nitrovinyl)furan nach Anspruch 3 zur Herstellung eines Arzneimittels zur Behandlung von Infektionen, die durch gramnegative Bakterien von Gattungen der Familie Pseudomonadaceae, wie Pseudomonas, verursacht werden.

5. Verwendung von 2-Brom-5-(2-brom-2-nitrovinyl)furan nach Anspruch 3 zur Herstellung eines Arzneimittels zur Behandlung von Infektionen, die durch gramnegative Bakterien von Gattungen der Familie Enterobacteriaceae, wie Escherichia, Proteus, Salmonella, Klebsiella, Serratia und Enterobacter, verursacht werden.

6. Verwendung von 2-Brom-5-(2-brom-2-nitrovinyl)furan nach Anspruch 3 zur Herstellung eines Arzneimittels zur Behandlung von Infektionen, die durch Bakterien von aeroben und mikroaerophilen Gattungen der Familie Brucellaceae, wie Pasteurella, Haemophilus und Moraxella, verursacht werden.

7. Verwendung von 2-Brom-5-(2-brom-2-nitrovinyl)furan nach Anspruch 3 zur Herstellung eines Arzneimittels zur Behandlung von Infektionen, die durch Bakterien von Gattungen der Familie Neisseriaceae, wie der Gattung Neisseria, verursacht werden.

8. Verwendung von 2-Brom-5-(2-brom-2-nitrovinyl)furan nach Anspruch 3 zur Herstellung eines Arzneimittels zur Behandlung von Infektionen, die durch Bakterien von Gattungen der Familie Micrococcaceae, wie Staphylococcus und Sarcina, verursacht werden.

9. Verwendung von 2-Brom-5-(2-brom-2-nitrovinyl)furan nach Anspruch 3 zur Herstellung eines Arzneimittels zur Behandlung von Infektionen, die durch Bakterien von Gattungen der Familie Lactobacillaceae, wie der Gattung Streptococcus, verursacht werden.

10. Verwendung von 2-Brom-5-(2-brom-2-nitrovinyl)furan nach Anspruch 3 zur Herstellung eines Arzneimittels zur Behandlung von Infektionen, die durch Gattungen asporogener grampositiver Bazillen der Familie Corynebacteriaceae, wie Corynebacterium und Listeria, verursacht werden.

11. Verwendung von 2-Brom-5-(2-brom-2-nitrovinyl)furan nach Anspruch 3 zur Herstellung eines Arzneimittels zur Behandlung von Infektionen, die durch Gattungen endosporogener grampositiver Bazillen der Familie Bacillaceae, wie der Gattung Bacillus, verursacht werden.

12. Verwendung von 2-Brom-5-(2-brom-2-nitrovinyl)furan nach Anspruch 3 zur Herstellung eines Arzneimittels zur Behandlung von Infektionen, die durch Gattungen der höheren Bakterien aus der Familie Treponemataceae, wie Leptospira, verursacht werden.

13. Verwendung von 2-Brom-5-(2-brom-2-nitrovinyl)furan nach Anspruch 3 zur Herstellung eines Arzneimittels zur Behandlung von Infektionen, die durch Gattungen der höheren Bakterien aus der Familie Spirillaceae, wie Vibrio, verursacht werden.

14. Verwendung von 2-Brom-5-(2-brom-2-nitrovinyl)furan nach Anspruch 3 zur Herstellung eines Arzneimittels zur Behandlung von Infektionen, die durch Scotobakterien aus der Familie Mycoplasmataceae verursacht werden.

15. Verwendung von 2-Brom-5-(2-brom-2-nitrovinyl)furan nach Anspruch 1 zur Herstellung eines Arzneimittels zur Behandlung von Infektionen, die sowohl durch momomorphe Sproßpilze als auch durch monomorphe Fadenpilze verursacht werden.

16. Verwendung von 2-Brom-5-(2-brom-2-nitrovinyl)furan nach Anspruch 15 zur Herstellung eines Arzneimittels zur Behandlung von Infektionen, die durch monomorphe Sproßpilze aus der Familie Cryptococcaceae, wie Candida und Malassezia, verursacht werden.

17. Verwendung von 2-Brom-5-(2-brom-2-nitrovinyl)furan nach Anspruch 15 zur Herstellung eines Arzneimittels zur Behandlung von Infektionen, die durch monomorphe Fadenpilze, darunter Dermatophyten, verursacht werden.

**18.** Verwendung von 2-Brom-5-(2-brom-2-nitrovinyl)furan nach Anspruch 15 zur Herstellung eines Arzneimittels zur Behandlung von Infektionen, die durch filamentöse monomorphe Gattungen aus der Familie Moniliaceae, wie Aspergillus, Trichophytum, Microsporum und Epidermophytum, verursacht werden.

**19.** Verwendung von 2-Brom-5-(2-brom-2-nitrovinyl)furan nach Anspruch 15 zur Herstellung eines Arzneimittels zur Behandlung von Infektionen, die durch filamentöse monomorphe Gattungen aus der Familie Tuberculariaceae, wie Fusarium sp., verursacht werden.

**20.** Pharmazeutische Zusammensetzung, die den Wirkstoff 2-Brom-5-(2-brom-2-nitrovinyl)furan nach Anspruch 1 in einer geeigneten Dosisform enthält.

**21.** Pharmazeutische Zusammensetzung nach Anspruch 20, die einen Träger, einen Grundstoff, ein Verdünnungsmittel oder einen Hilfsstoff enthält.

**Revendications**

**1.** 2-bromo-5-(2-bromo-2-nitro-vinyl)-furane à utilisation médicamenteuse en médecine humaine et vétérinaire.

**2.** 2-bromo-5-(2-bromo-2-nitro-vinyl)-furane selon la revendication 1, où la pureté de ce composé est d'au moins 98 %.

**3.** Utilisation du 2-bromo-5-(2-bromo-2-nitro-vinyl)-furane pour la fabrication d'un médicament destiné à traiter des infections microbiennes provoquées par des bactéries Gram négatives, Gram positives, des bactéries supérieures et des scotobactéries.

**4.** Utilisation du 2-bromo-5-(2-bromo-2-nitro-vinyl)-furane selon la revendication 3, pour la fabrication d'un médicament destiné à traiter des infections provoquées par des bactéries Gram négatives du genre de la famille des Pseudomonadaceae comme Pseudomonas.

**5.** Utilisation du 2-bromo-5-(2-bromo-2-nitro-vinyl)-furane selon la revendication 3, pour la fabrication d'un médicament destiné à traiter des infections provoquées par des bactéries Gram négatives du genre de la famille des Enterobacteriaceae comme Escherichia, Proteus, Salmonella, Klebsiella, Serratia et Enterobacter.

**6.** Utilisation du 2-bromo-5-(2-bromo-2-nitro-vinyl)-furane selon la revendication 3, pour la fabrication d'un médicament destiné à traiter des infections provoquées par les genres aérobie et aérophile de la famille des Brucelaceae comme Pasteurella, Haemophilus et Moraxella.

**7.** Utilisation du 2-bromo-5-(2-bromo-2-nitro-vinyl)-furane selon la revendication 3, pour la fabrication d'un médicament destiné à traiter des infections provoquées par des bactéries du genre de la famille des Neisseriaecae comme le genre Neisseria.

**8.** Utilisation du 2-bromo-5-(2-bromo-2-nitro-vinyl)-furane selon la revendication 3, pour la fabrication d'un médicament destiné à traiter des infections provoquées par des bactéries du genre de la famille des Micrococcaceae comme Staphylococcus et Sarcina.

**9.** Utilisation du 2-bromo-5-(2-bromo-2-nitro-vinyl)-furane selon la revendication 3, pour la fabrication d'un médicament destiné à traiter des infections provoquées par des bactéries du genre de la famille des Lactobacillaceae comme le genre Streptococcus.

**10.** Utilisation du 2-bromo-5-(2-bromo-2-nitro-vinyl)-furane selon la revendication 3, pour la fabrication d'un médicament destiné à traiter des infections provoquées par le genre de bacilles Gram positives asporogènes de la famille des Corynebacteriaceae comme Corynebacterium et Listeria.

**11.** Utilisation du 2-bromo-5-(2-bromo-2-nitro-vinyl)-furane selon la revendication 3, pour la fabrication d'un médicament destiné à traiter des infections provoquées par le genre de bacilles Gram positives endosporogènes de la famille des Bacillaceae comme le genre Bacillus.

**12.** Utilisation du 2-bromo-5-(2-bromo-2-nitro-vinyl)-furane selon la revendication 3, pour la fabrication d'un médicament destiné à traiter des infections provoquées par le genre de bactéries supérieures de la famille des Treponemataceae comme Leptospira.

**13.** Utilisation du 2-bromo-5-(2-bromo-2-nitro-vinyl)-furane selon la revendication 3, pour la fabrication d'un médicament destiné à traiter des infections provoquées par le genre de bactéries supérieures de la famille des Spirillaceae comme Vibrio.

**14.** Utilisation du 2-bromo-5-(2-bromo-2-nitro-vinyl)-furane selon la revendication 3, pour la fabrication d'un médicament destiné à traiter des infections provoquées par des scotobactéries de la famille des Mycoplasmataceae.

**15.** Utilisation du 2-bromo-5-(2-bromo-2-nitro-vinyl)-furane selon la revendication 1, pour la fabrication d'un médicament destiné à traiter des infections provoquées par le bourgeonnement de champignons monomorphes et de champignons monomorphes filamenteux.

**16.** Utilisation du 2-bromo-5-(2-bromo-2-nitro-vinyl)-furane selon la revendication 15, pour la fabrication d'un médicament destiné à traiter des infections provoquées par le bourgeonnement de champignons monomorphes de la famille des Cryptococcaceae comme Candida et Malassezia.

**17.** Utilisation du 2-bromo-5-(2-bromo-2-nitro-vinyl)-furane selon la revendication 15, pour la fabrication d'un médicament destiné à traiter des infections provoquées par des champignons monomorphes filamenteux y compris les dermatophytes.

**18.** Utilisation du 2-bromo-5-(2-bromo-2-nitro-vinyl)-furane selon la revendication 15, pour la fabrication d'un médicament destiné à traiter des infections provoquées par le genre monomorphe filamenteux de la famille des Moniliaceae comme Aspergillus, Tricophytum, Microsporum et Epidermophytum.

**19.** Utilisation du 2-bromo-5-(2-bromo-2-nitro-vinyl)-furane selon la revendication 15, pour la fabrication d'un médicament destiné à traiter des infections provoquées par le genre monomorphe filamenteux de la famille des Tuberculariaceae comme l'espèce Fusarium.

**20.** Composition pharmaceutique contenant la substance active 2-bromo-5-(2-bromo-2-nitro-vinyl)-furane selon la revendication 1, sous une forme de dosage appropriée.

**21.** Composition pharmaceutique de la revendication 20, où la composition contient un porteur, un excipient, un diluant ou un adjuvant.